Europäisches Patentamt

European Patent Office

Office européen des brevets

(19)

(11) Veröffentlichungsnummer: **0 066 092**
**B1**

(12) **EUROPÄISCHE PATENTSCHRIFT**

(45) Veröffentlichungstag der Patentschrift:
31.07.85

(51) Int. Cl.⁴: **A 61 F 2/34**

(21) Anmeldenummer: **82103593.8**

(22) Anmeldetag: **28.04.82**

(54) Prothesenschale für die Acetabulumpfanne eines Hüftgelenkes.

(30) Priorität: 30.05.81 DE 8116140 U
22.07.81 DE 8121441 U

(43) Veröffentlichungstag der Anmeldung:
08.12.82 Patentblatt 82/49

(45) Bekanntmachung des Hinweises auf die Patenterteilung:
31.07.85 Patentblatt 85/31

(84) Benannte Vertragsstaaten:
AT BE CH DE FR GB IT LI NL

(56) Entgegenhaltungen:
DE - A - 2 247 721
DE - A - 2 340 734
DE - A - 2 411 617
FR - A - 2 210 909
FR - A - 2 266 491
GB - A - 1 126 961
GB - A - 1 325 534

(73) Patentinhaber: **orthoplant Endoprothetik GmbH,**
**Leerkämpe 12, D-2800 Bremen 1 (DE)**

(72) Erfinder: **Schelhas, Klaus-Dieter, Semperstrasse 3,**
**D-2000 Hamburg 60 (DE)**
Erfinder: **Rusdea, Johan, Schillerstrasse 6,**
**D-2800 Bremen 1 (DE)**

(74) Vertreter: **Eisenführ & Speiser, Martinistrasse 24,**
**D-2800 Bremen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die Erfindung betrifft eine Prothesenschale für die Acetabulumpfanne eines Hüftgelenkes zur Lagerung eines insbesondere künstlichen Femurkopfes, mit einer an ihrer Außenseite im Pfannengewebe zu befestigenden Außenschale und einer an ihrer Innenseite auf dem Femurkopf zu lagernden, formschlüssig mit der Außenschale verbundenen Innenschale sowie einer zwischen der Außenschale und der Innenschale angeordneten Pufferschicht.

Zur chirurgischen Behandlung von Oberschenkelfrakturen, Arthrose u. dgl. sind Hüftgelenk-Endoprothesen mit einer im Acetabulumgewebe eines zu behandelnden Hüftgelenkes zu verankernden, kappenförmigen Prothesenschale bekannt, die an ihrer Außen- und Innenseite in aller Regel im wesentlichen kugelkalottenförmig ausgebildet sind und aus einem gewebeverträglichen Kunststoff oder einem anderen gewebeverträglichen Material bestehen, wobei die Befestigung im Acetabulumgewebe je nach Ausgestaltung der Prothesenschale entweder mit Knochenzement oder auch zementfrei erfolgen kann.

Nachdem man sich zunächst den mit der erforderlichen Gewebeverträglichkeit, der Lager- bzw. Laufeigenschaften, des Abriebs o. dgl. zusammenhängenden Materialfragen und den Verankerungsproblemen zugewandt und diese weitgehend befriedigend gelöst hatte, hat man versucht, Hüftgelenkprothesen zu entwickeln, die den natürlichen Hüftgelenken im Hinblick auf die Elastizität und Dämpfung des Knochengewebes besser entsprechen als dieses bei Hüftgelenkprothesen mit weitgehend starrer Prothesenschale der Fall war.

So ist aus der DE-A1-2 411 617 eine Prothesenschale der eingangs beschriebenen Gattung bekanntgeworden, mit welcher zwar in erster Linie die Reibung zwischen dem im wesentlichen kugelförmigen Femurkopf und der Prothesenschale herabgesetzt, darüber hinaus aber auch eine gewisse Stoßdämpfung erzielt werden sollte. Bei dieser bekannten Prothesenschale besteht die kalottenförmige Innenschale aus Keramik und ist an ihrer der Außenschale zugekehrten Außenseite mit einer Kunststoffummantelung versehen, wobei eine formschlüssige Verbindung zwischen der Innenschale und der Außenschale dadurch hergestellt ist, daß die Kunststoffummantelung an der Innenseite ihres Randes einen radial nach innen vorstehenden Flansch aufweist, an dem die Innenschale gehalten ist, während die Außenschale einen entsprechenden Flansch aufweist, welcher die Kunststoffummantelung der Innenschale hält, so daß mithin zwei Schnappverbindungen vorliegen, welche die drei Schichten der Prothesenschale in formschlüssiger Verbindung halten.

Es hat sich indes gezeigt, daß mit dieser bekannten Prothesenschale zwar verhältnismäßig gute Laufeigenschaften bezüglich Reibung und Abrieb zu erzielen sind, daß aber die elastischen bzw. dämpfenden Eigenschaften noch mangelhaft sind, und daß man mithin kaum von einer beachtlichen Pufferwirkung der Kunststoffummantelung der Innenschale und damit der Gesamtprothesenschale sprechen kann. Dieses liegt u. a. daran, daß schon das Verformungsvermögen des gewählten Materials bei den auftretenden Belastungen sehr gering ist, wobei dieses durch die konstruktive Ausgestaltung dieser bekannten Prothesenschale noch unterstützt wird.

Aus der FR-A-2 210 909 ist eine Prothesenschale bekanntgeworden, die gegenüber der vorstehend beschriebenen Prothesenschale hinsichtlich ihrer elastischen und dämpfenden Puffereigenschaften insoweit verbessert ist, als für das Material der zwischen der Außen- und Innenschale angeordneten Pufferschicht Silikon verwendet wird, welches erheblich bessere Puffereigenschaften aufweist. Es hat sich jedoch gezeigt, daß es bei einer Belastung dieser bekannten Prothesenschale, bei welcher die Pufferschicht aus einer bis zum Rand durchgehenden Silikonschicht besteht, zu einem Herausquetschen von Silikon am Schalenrand kommen kann, zumal die Verformungsmöglichkeit der Silikonschicht in den weiter vom Rand entfernt liegenden Abschnitten durch die mögliche Materialkompression begrenzt ist und kein Ausweichen in irgendwelche Verformungsräume zuläßt. Dieses gilt gleichermaßen für die aus der DE-A-2 247 721 bekanntgewordene Prothesenschale.

Der vorliegenden Erfindung liegt die Aufgabe zugrunde, die bekannten Prothesenschalen der eingangs beschriebenen Gattung so zu verbessern, daß eine den Puffereigenschaften eines natürlichen Hüftgelenkes zumindest weitgehend entsprechende elastische bzw. dämpfende Pufferung gegeben ist, ohne daß es aufgrund der Pufferschicht zu den bisherigen Nachteilen wie einem Herausquetschen des Materials der Pufferschicht o. dgl. kommt.

Die Lösung dieser Aufgabe ist erfindungsgemäß dadurch gekennzeichnet, daß die in an sich bekannter Weise aus Silikon bestehende Pufferschicht mehrere mit gegenseitigem Abstand angeordnete Durchgangslöcher aufweist, daß die Pufferschicht im nicht belasteten Ruhezustand des Gelenkes mit Abstand zu einer Schulterfläche einer von der Außenseite des unteren Randabschnittes der Innenschale vorstehenden Schultern endet, und daß die Innenschale an dem ihrem freien Rand benachbarten Abschnitt ihrer Außenseite einen Vorsprung aufweist, der in eine an der Innenseite der Außenschale vorgesehene nutförmige Ausnehmung greift.

Bei der erfindungsgemäßen Prothesenschale ist die aus dem für eine solche Pufferung hervorragend geeigneten Silikon bestehende Pufferschicht mithin praktisch völlig zwischen der Außen- und der Innenschale eingekapselt, so daß sie schon deswegen nicht am unteren Rand der Prothesenschale bei Belastung aus dieser ausgequetscht werden kann, wobei darüber hinaus mehrere Maßnahmen getroffen worden sind, um der Pufferschicht bei Belastung und entsprechender Verformung Ausweichmöglichkeiten zu geben. Diese Maßnahmen bestehen zum einen darin, daß die Pufferschicht bei Belastung nicht nur komprimiert wird, sondern sich gleichsam darüber hinaus in die vorzugsweise verteilt über die Pufferschicht angeordneten Durchgangslöcher hinein verformen kann. Hierdurch wird der erstrebte Puffereffekt ganz erheblich verbessert und zugleich sichergestellt, daß die Verformung zum Rand der Prothesenschale hin entsprechend verkleinert wird. Wenn sich in den Durchgangslöchern Körperflüssigkeit angesammelt hat, wird diese mit einer gewissen zeitlichen Verzögerung bei Belastung der Pufferschicht aus den Löchern gedrückt und kann über die formschlüssige Verbindung zwischen der Außen- und Innenschale austreten, was die Gebrauchseigenschaft ebenfalls noch verbessert.

Eine weitere Maßnahme zur Verbesserung der Puffereigenschaften und zur Verhinderung eines Herausquetschens der Pufferschicht aus dem Rand der Prothesenschale besteht darin, daß sich die Pufferschicht erfindungsgemäß nicht bis zum Rand hin frei erstreckt, sondern mit Abstand zum Rand der Prothesenschale endet, und zwar darüber hinaus noch mit einem Abstand zu einer Schulter der Innenschale, so daß bei Belastung zunächst eine Verformung der Pufferschicht in Richtung auf die Schulter in den freien Raum hinein möglich ist und es sodann zur Anlage der Stirnseite der Pufferschicht an der Schulterfläche kommt, so daß ein Herausquetschen von Silikonmaterial ausgeschlossen ist.

Die vorgenannten Maßnahmen in Verbindung mit der abweichend vom vorbekannten Stand der Technik ausgebildeten, unmittelbar formschlüssigen Verbindung zwischen der Außen- und der Innenschale in der Art einer Nut-Feder-Verbindung — welche zugleich die bereits weiter oben erwähnte Drainage von Körperflüssigkeit ermöglicht — ergeben insgesamt eine optimale Pufferwirkung unter Ausschaltung sämtlicher bisher bekannter nachteiliger Effekte, wobei zugleich eine sehr einfache Montagemöglichkeit gegeben ist.

Bei einer besonders zweckmäßigen Ausgestaltung der vorliegenden Erfindung besteht der in die nutförmige Ausnehmung der Außenschale eingreifende Vorsprung der Innenschale aus einem an deren Außenseite eingesetzten, bevorzugt in radialer Richtung geschlitzen Ring, der vorzugsweise aus einem Edelmetall bzw. Edelmetallegierung besteht und in eine ihm entsprechende nutförmige Ausnehmung an der Außenseite der Innenschale eingesetzt sein kann, wobei eine solche Ausgestaltung nicht nur den auftretenden Beanspruchungen und der erforderlichen Lebensdauer der Prothese in optimaler Weise Rechnung trägt, sondern darüber hinaus auch bezüglich der Herstellung und der Montage als besonders vorteilhaft anzusehen ist.

Es sei noch darauf verwiesen, daß statt einer einzigen Pufferschicht auch mehrere Pufferschichten vorgesehen sein können, wobei in einem solchen Falle jeweils zwischen zwei einander benachbarten Pufferschichten eine Zwischen-Stützschicht angeordnet sein kann, die bevorzugt aus dem gleichen Material besteht wie die Innenschale und/oder die Außenschale.

Bevorzugte Ausgestaltungen der vorliegenden Erfindung sind in den abhängigen Ansprüchen beschrieben.

Die Erfindung ist nachstehend an einem Ausführungsbeispiel unter Bezugnahme auf eine Zeichnung weiter erläutert. Es zeigt

Fig. 1 eine Seitenansicht einer erfindungsgemäßen Prothesenschale in Richtung des Pfeiles I in Fig. 2 gesehen;

Fig. 2 eine Draufsicht auf die Prothesenschale gemäß Fig. 1 in Richtung des Pfeiles II in Fig. 1 gesehen;

Fig. 3 eine Draufsicht von unten auf die Prothesenschale gemäß den Fig. 1 und 2 in Richtung des Pfeiles III in Fig. 1 gesehen;

Fig. 4 einen Schnitt durch die Prothesenschale gemäß den Fig. 1 bis 3 in Richtung der Schnittlinie IV-IV in Fig. 2 gesehen;

Fig. 5 den in Fig. 4 mit einem Vollkreis eingerahmten und mit V bezeichneten Abschnitt in vergrößerter Darstellung;

Fig. 6 eine Draufsicht auf die Pufferschicht der Prothesenschale gemäß den Fig. 1 bis 5 in Richtung des Pfeiles VI in Fig. 7 gesehen;

Fig. 7 einen Schnitt durch die Pufferschicht gemäß Fig. 6 der Prothesenschale gemäß den Fig. 1 bis 5 in Richtung der Schnittlinie VII-VII gesehen;

Fig. 8 eine Fig. 4 entsprechende Variante, bei welcher der Vorsprung der Innenschale als geschlitzter Metallring ausgebildet ist;

Fig. 9 den in Fig. 8 mit einem Kreis umrandeten Abschnitt in vergrößerter Darstellung, wobei die Außenseite der Außenschale nicht profiliert ist;

Fig. 10 einen mittleren Schnitt durch die Außenschale der Prothesenschale gemäß den Fig. 9;

Fig. 11 einen mittleren Schnitt durch die Pufferschicht der Prothesenschale gemäß den Fig. 8 und 9; und

Fig. 12 einen mittleren Schnitt durch die Innenschale der Prothesenschale gemäß Fig. 9.

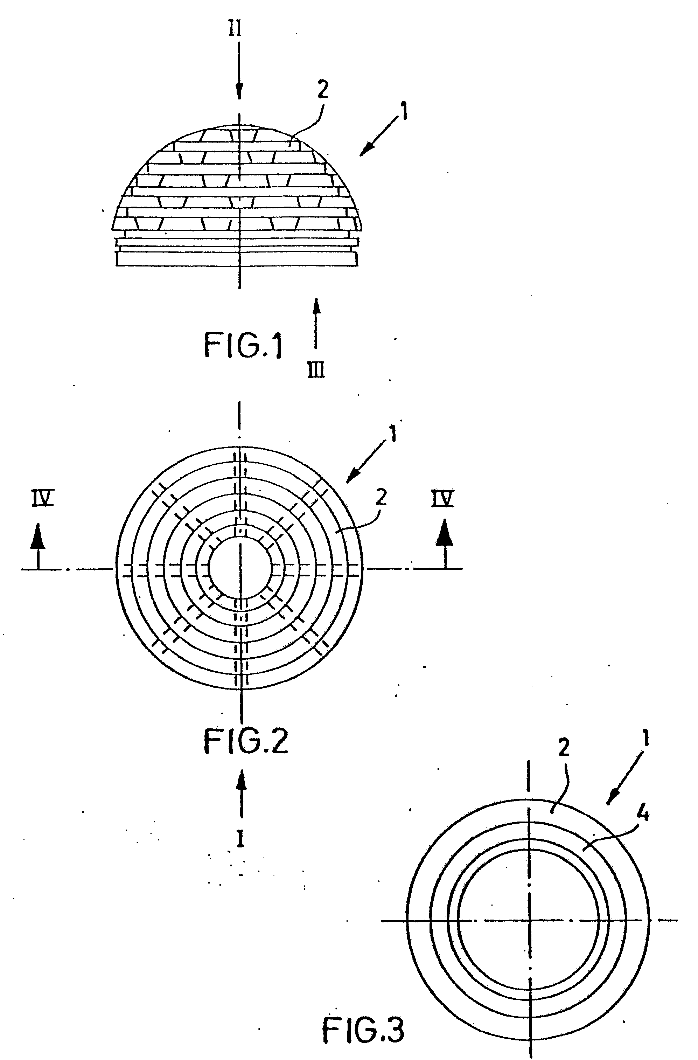

Die Fig. 1—7 zeigen eine im ganzen mit 1 bezeichnete Prothesenschale für die Acetabulumpfanne eines in der Zeichnung nicht dargestellten Hüftgelenkes als Lagerschale für den künstlichen Femurkopf einer Total-Endoprothese.

Die Prothesenschale 1 weist eine Außenschale 2 aus Polyäthylen auf, die mit ihrer Außenseite 3 dem natürlichen Knochengewebe der Acetabulumpfanne zugekehrt ist, welches vor dem Einpflanzen der Prothesenschale 1 durch im wesentlichen kugelförmige Ausfräsung entsprechend vorbereitet wird.

Außerdem besitzt die Prothesenschale 1 eine mit der Außenschale 2 auf noch zu beschreibende Weise verbundene Innenschale 4, deren Innenseite 6 im eingepflanzten Zustand dem nicht dargestellten Femurkopf zugekehrt ist.

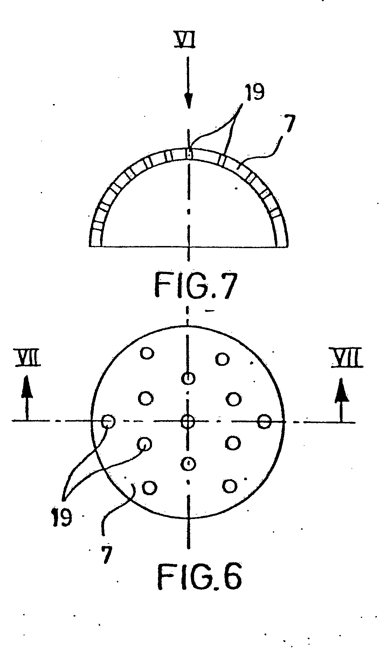

Zwischen der Außenschale 2 und der Innenschale 4 ist eine aus Silikon bestehende Pufferschicht 7 vorhanden (siehe auch Fig. 6 und 7), die elastische und dämpfende Eigenschaften besitzt.

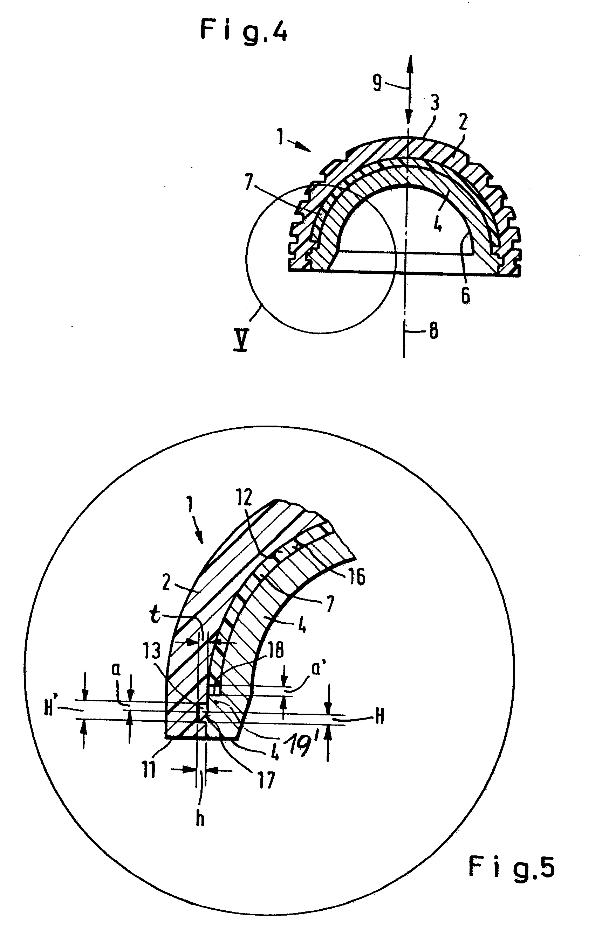

Die Außenschale 2 und die Innenschale 4 sind durch das Vorhandensein der Pufferschicht 7 sowie durch Maßnahmen, die weiter unten noch beschrieben sind, in Richtung des auf der Symmetrieachse 8 liegenden Doppelpfeils 9 (Fig. 4) relativ zueinander beweglich, und zwar sind sie zu diesem Zweck so formschlüssig miteinander verbunden, daß die Innenschale 4 im nicht belasteten Ruhezustand des Gelenkes in einem vorgegebenen Abstand a von 2 bis 3 mm zur Außenschale 2 steht und sich demgemäß um etwa dieses Maß relativ zu jener bewegen kann.

Hierfür besitzt die Außenschale 2 an ihrem ihrem freien Rand 11 benachbarten Abschnitt ihrer Innenseite 12 eine umlaufende, nutförmige Ausnehmung 13.

Weiterhin besitzt zu diesem Zwecke die Innenschale 4 an ihrem freien Rand 14 benachbarten Abschnitt ihrer Außenseite 16 einen der nutförmigen Ausnehmung 13 der Außenschale 2 angepaßten Vorsprung 17, der mit der nutförmigen Ausnehmung 13 formschlüssig zu verbinden ist, wie dieses insbesondere aus Fig. 5 erkennbar ist.

Dabei besitzt die nutförmige Ausnehmung 13 in einem Radialschnitt (siehe Fig. 4, 5) eine rechteckige Form, während der Vorsprung 17 der Innenschale 4 in einem Radialschnitt dreiecksförmig ausgebildet ist, wobei die radiale Höhe h des Vorsprungs 17 geringfügig kleiner ist als die radiale Tiefe t der nutförmigen Ausnehmung 13, und wobei vor allem auch die in Richtung des Pfeiles 9 gemessene Höhe H des Vorsprungs 17 kleiner ist als die Höhe H' der Ausnehmung 13.

Wie insbesondere aus Fig. 5 ersichtlich ist, besitzt die Innenschale 4 benachbart zu dem Vorsprung 17 an ihrer Außenseite 16 eine Schulter, deren Schulterfläche 18 im Ruhezustand mit Abstand a' zur Stirnseite der Pufferschicht 7 angeordnet ist.

Wie aus den Fig. 6 und 7 hervorgeht, kann die Pufferschicht 7 mehrere mit gegenseitigem Abstand angeordnete und vorzugsweise über die gesamte Oberfläche gleichmäßig verteilte Durchgangslöcher 19 aufweisen.

Anders als bei dem in der Zeichnung dargestellten Ausführungsbeispiel können zur Lösung der der vorliegenden Erfindung zugrunde liegenden Aufgabe auch mehrere Pufferschichten 7 vorgesehen sein, wobei bei einer derartigen Ausgestaltung bevorzugt dann jeweils zwischen zwei einander benachbarten Pufferschichten eine Zwischen-Stützschicht angeordnet ist, die dann zweckmäßigerweise aus dem gleichen Material besteht wie die Innenschale 4 und die Außenschale 2.

Während die Zeichnung (siehe insbesondere Fig. 4 und 5) den weiter oben definierten Ruhezustand der erfindungsgemäßen Prothesenschale 1 darstellt, ist erkennbar, daß bei Belastung der Außenschale 2 in Richtung auf die Innenschale 4 oder umgekehrt eine elastische und zugleich dämpfende Verformung der Pufferschicht 7 auftritt. Dabei wird die Verformung der Pufferschicht 7 in radialer Richtung dadurch unterstützt, daß die Pufferschicht 7 an ihrem Rand bzw. ihrer Stirnseite mit einem Abstand a' zur Schulterfläche 18 endet, so daß bei der vorstehend beschriebenen Verformung in radialer Richtung eine »Umfangsverformung« (Längung) in den zwischen der Schulterfläche 18 und der Stirnfläche (Rand) der Pufferschicht 7 vorhandenen freien Raum stattfinden kann. Es ist jedoch erkennbar, daß auch das sich in diesem freien Raum unter Belastung hineinverformende Material der Pufferschicht 7 bei weitersteigender Belastung verformbar ist, so daß eine solche Konstruktion insgesamt eine elastische Kennlinie mit progressiver Federkennzahl aufweist, wie dieses auch bei einem natürlichen Hüftgelenk der Fall ist.

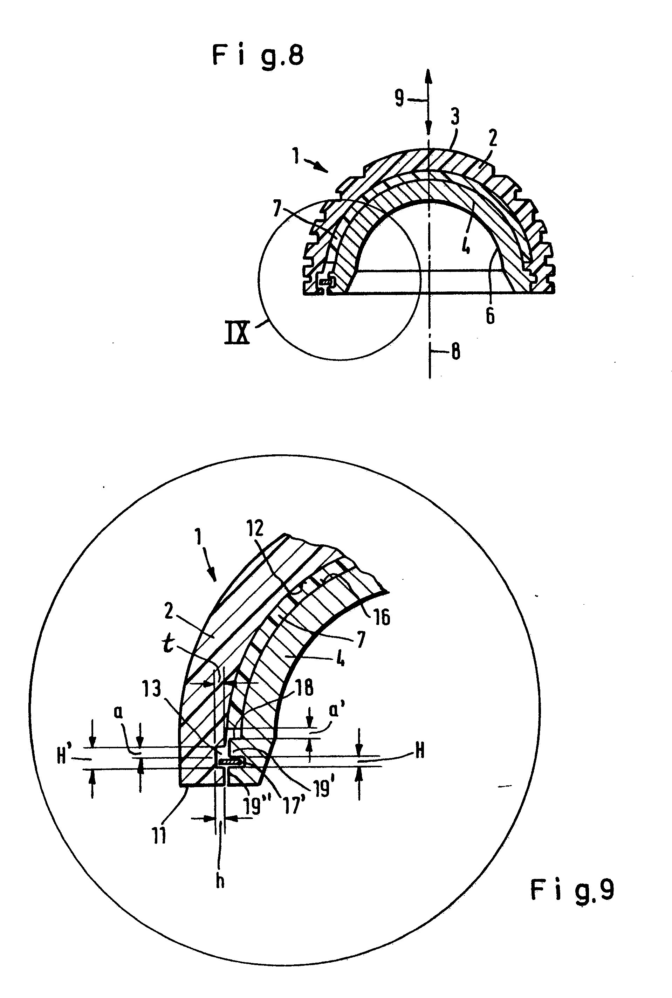

Die Fig. 8—12 der Zeichnung zeigen eine Variante der Ausgestaltung gemäß den Fig. 1 bis 7, bei welcher der in die nutförmige Ausnehmung 13 der Außenschale 2 eingreifende Vorsprung der Innenschale 4 nicht integral mit der Innenschale 4 ausgebildet ist, sondern aus einem in die Innenschale 4 eingesetzen Ring 17' besteht. Der Ring 17' besitzt einen im wesentlichen rechteckigen Querschnitt und ist an seinem der Pufferschicht 7 zugekehrten freien oberen Rand abgeschrägt. Er besitzt einen im wesentlichen radial verlaufenden, in der Zeichnung nicht dargestellten, durchgehenden Schlitz, um ihn an der Innenschale 4 montieren zu können, und besteht aus Titan. Der Ring 17' ist in eine nutförmige Ausnehmung 17'' an der Außenseite der Innenschale 4 eingesetzt und steht über diese nach außen vor. Die nutförmige Ausnehmung 17'' zur Aufnahme des Ringes 17' wird von zwei an der Außenseite der Innenschale 4 vorstehenden Vorsprüngen 19' und 19'' gebildet.

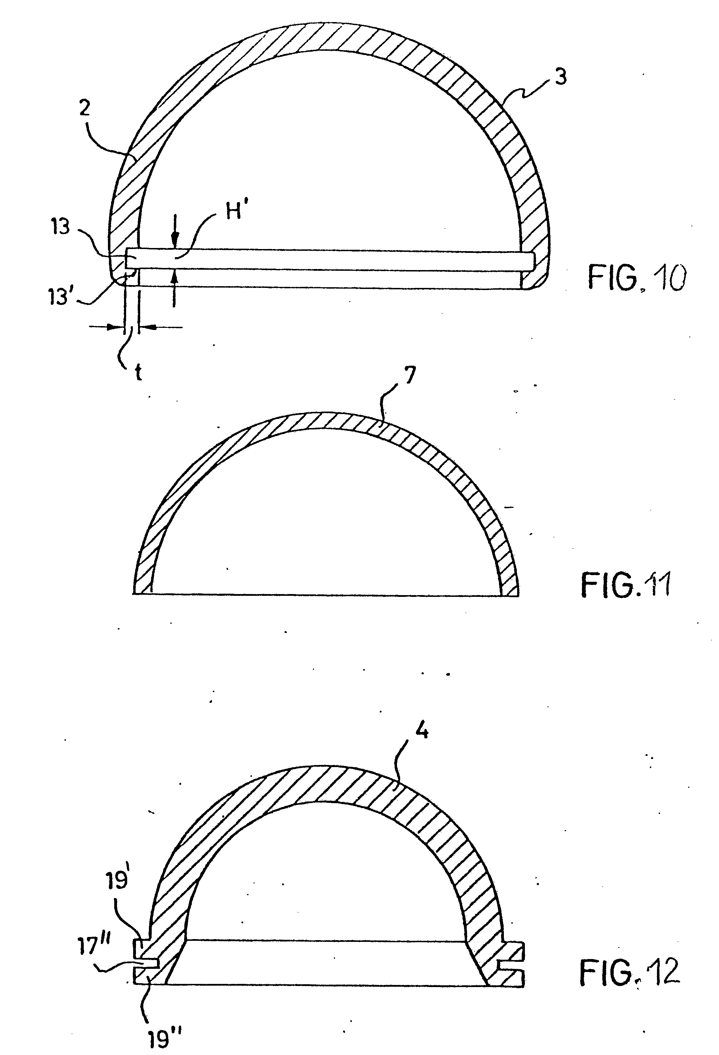

Die über die Außenfläche der Innenschale 4 vorstehende radiale Höhe h des Ringes 17' ist kleiner als die radiale Tiefe t der nutförmigen Ausnehmung 13 der Außenschale. Ebenfalls ist die in Richtung des Pfeiles 9 gemessene Höhe H des Ringes 17' kleiner als die Höhe H' der Ausnehmung 13 (Fig. 9, 10).

Um die Innenschale 4 mit dem Ring 17' in die in Fig. 9 gezeigte Verankerungsstellung zu bringen, hat es sich als zweckmäßig erwiesen, die Außenschale 2 beispielsweise in heißem Wasser zu erwärmen und die aufgrund der Temperaturerhöhung ausgedehnte Außenschale 2 in erwärmtem Zustand über die Innenschale 4 und damit den Ring 17' zu drücken. Dabei gleitet der Ring 17' aufgrund seiner Abschrägung ohne Schwierigkeiten über den Innenrand des freien Randes 11 der Außenschale in die Ausnehmung 13.

Insgesamt ergibt die erfindungsgemäße Prothesenschale gegenüber dem bisher bekannten Stand der Technik eine unglaubliche Verbesserung und kann bei besonders zweckmäßiger Auslegung zu einer Prothese führen, deren Eigenschaften den entsprechenden Eigenschaften des natürlichen Gelenkes sogar überlegen sind, wobei abschließend noch einmal erwähnt wird, daß die erfindungsgemäße Prothesenschale ersichtlich nicht allein, wenngleich insbesondere, für Hüftgelenke bestimmt und geeignet ist, sondern auch für andere Gelenkprothesen oder Teilprothesen.

**Patentansprüche**

1. Prothesenschale für die Acetabulumpfanne eines Hüftgelenkes zur Lagerung eines insbesondere künstlichen Femurkopfes, mit einer an ihrer Außenseite (3) im Pfannengewebe zu befestigenden Außenschale (2) und einer an ihrer Innenseite (12) auf dem Femurkopf zu lagernden, formschlüssig mit der Außenschale (2) verbundenen Innenschale (4) sowie einer zwischen der Außenschale (2) und der Innenschale (4) angeordneten Pufferschicht (7), dadurch gekennzeichnet, daß die in an sich bekannter Weise aus Silikon bestehende Pufferschicht (7) mehrere mit gegenseitigem Abstand angeordnete Durchgangslöcher (19) aufweist; daß die Pufferschicht (7) im nicht belasteten Ruhezustand des Hüftgelenkes mit Abstand (a') zu einer Schulterfläche (18) einer von der Außenseite (16) des unteren Randabschnittes der Innenschale (4) vorstehenden Schultern (19) endet; und daß die Innenschale (4) an dem ihrem freien Rand (14) benachbarten Abschnitt ihrer Außenseite (16) einen Vorsprung (17; 17') aufweist, der in eine an der Innenseite (12) der Außenschale (2) vorgesehene nutförmige Ausnehmung (13) greift.

2. Prothesenschale nach Anspruch 1, dadurch gekennzeichnet, daß die nutförmige Ausnehmung (13) einen im wesentlichen rechtwinkligen radialen Querschnitt besitzt; und daß der Vorsprung (17) der Innenschale (4) dreieckförmig ausgebildet ist, wobei die radiale Erstreckung (h) des Vorsprunges (17) etwa gleich der radialen Tiefe (t) der nutförmigen Ausnehmung (13) ist, und wobei die Höhe (H) des Vorsprunges (17) kleiner ist als die Höhe (H') der Ausnehmung (13).

3. Prothesenschale nach Anspruch 1, dadurch gekennzeichnet, daß der in die nutförmige Ausnehmung (13) der Außenschale (2) greifende Vorsprung der Innenschale (4) aus einem in die Innenschale (4) eingesetzten Ring (17') besteht.

4. Prothesenschale nach Anspruch 3, dadurch gekennzeichnet, daß der einen im wesentlichen rechtwinkligen Querschnitt aufweisende Ring (17') an seinem freien oberen Rand abgeschrägt ist.

5. Prothesenschale nach Anspruch 3 oder 4, dadurch gekennzeichnet, daß der Ring (17') mit einem im wesentlichen radial verlaufenden Durchgangsschlitz versehen ist.

6. Prothesenschale nach einem oder mehreren der Ansprüche 3 bis 5, dadurch gekennzeichnet, daß der Ring (17') aus einem Edelmetall bzw. einer Edelmetallegierung besteht.

7. Prothesenschale nach einem oder mehreren der Ansprüche 3 bis 5, dadurch gekennzeichnet, daß der Ring (17') aus Titan besteht.

8. Prothesenschale nach einem oder mehreren der Ansprüche 3 bis 7, dadurch gekennzeichnet, daß der Ring (17') in einer nutförmigen Ausnehmung (17'') an der Außenseite (16) der Innenschale (4) angeordnet ist.

9. Prothesenschale nach einem oder mehreren der Ansprüche 3 bis 8, dadurch gekennzeichnet, daß die nutförmige Ausnehmung (17'') zur Aufnahme des Ringes (17') von zwei an der Außenseite (16) der Innenschale (4) vorstehenden Schultern (19', 19'') gebildet ist.

10. Prothesenschale nach einem oder mehreren der vorhergehenden Ansprüche, dadurch gekennzeichnet, daß mehrere Pufferschichten (7) vorgesehen sind.

11. Prothesenschale nach Anspruch 10, dadurch gekennzeichnet, daß jeweils zwischen zwei einander benachbarten Pufferschichten (7, 7) eine Zwischen-Stützschicht angeordnet ist.

12. Prothesenschale nach Anspruch 11, dadurch gekennzeichnet, daß eine Zwischen-Stützschicht aus dem gleichen Material besteht wie die Innenschale (4) oder/und die Außenschale (2).

**Claims**

1. Prothesis shell for the acetabulum cup of a hip joint for the mounting of an in particular synthetic femur head, comprising an outer shell (2) the outside (3) of which is to be fixed in the cup tissue and an inner shell (4) which is positively connected to the outer shell (2) and the inside (12) of which is to be mounted on the femur head, and further comprising a cushioning layer (7) disposed between the outer shell (2) and the inner shell (4), characterised in that the cushioning layer (7) consisting in per se known manner of silicone has a plurality of mutually spaced apart holes (19) traversing it, and in that when the hip joint is in the resting state and is not under load the cushioning layer (7) ends at a distance (a') from a shoulder face (18) of one of the shoulders (19) protruding from the outside (16) of the bottom edge portion of the inner shell (4) and in that the inner shell (4) has on the portion of its outside (16) neighbouring on its free edge (14) a projection (17, 17') which engages into a slot-shaped recess (13) provided on the inside (12) of the outer shell (2).

2. Prothesis shell according to Claim 1, characterised in that the slot-shaped recess (13) has a substantially rectangular radial cross-section and in that the projection (17) on the inner shell (4) ist of triangular shape, the radial extention (h) of the projection (17) being approximately equal to the radial depth (t) of the slot-shaped recess (13) and the height (H) of the projection (17) being smaller than the height (H') of the recess (13).

3. Prothesis shell according to Claim 1, characterised in that the projection on the inner shell (4) which engages into the slot-shaped recess (13) of the outer shell (2) consists of a ring (17') inserted into the inner shell (4).

4. Prothesis shell according to Claim 3, characterised in that the ring (17') which is of substantially rectangular cross section is chamfered on its free upper edge.

5. Prothesis shell according to Claim 3 or 4, characterised in that the ring (17') is provided with a substantially radially extending continuous slot.

6. Prothesis shell according to one or more of Claims 3 to 5, characterised in that the ring (17') consists of a rare metal or a rare metal alloy.

7. Prothesis shell according to one or more of Claims 3 to 5, characterised in that the ring (17') consists of titanium.

8. Prothesis shell according to one or more of Claims 3 to 7, characterised in that the ring (17') is disposed in a groove-like recess (17'') on the outside (16) of the inner shell (4).

9. Prothesis shell according to one or more of Claims 3 to 8, characterised in that the groove-like recess (17'') for accommodating the ring (17') is formed by two shoulders (19', 19'') protruding from the outside (16) of the inner shell (4).

10. Prothesis shell according to one or more of the pre eding Claims, characterised in that a plurality of cushioning layers (7) are provided.

11. Prothesis shell according to Claim 10, characterised in that an intermediate bracing layer is disposed between every two adjacently disposed cushioning layers (7, 7).

12. Prothesis shell according to Claim 11, characterised in that an intermediate bracing layer consists of the same material as the inner shell (4) and/or the outer shell (2).

## Revendications

1. Cupule prothétique pour la cavité cotyloïde d'une articulation be hanche destinée à recevoir une tête de fémur, notamment artificielle, avec une cuvette externe (2) se fixant par sa face extérieure (3) dans le tissu de la cavité et une cuvette interne (4) se montant par sa face intérieure (12) sur la tête de fémur et assemblée par liaison géométrique avec la cuvette externe (2), ainsi qu'une couche tampon (7) disposée entre la cuvette externe (2) et la cuvette interne (4), caractérisée en ce que la couche tampon (7), constituée en silicone de façon connue en soi, présente plusieurs perçages traversants (19) espacés les uns des autres et en ce qu'à l'état de repos, non chargé, de l'articulation de hanche, la couche tampon (7) se termine à distance (a') d'une face d'épaulement (18) de l'un des épaulements (19') en saillie sur la face externe (14) de la partie de bordure inférieure de la cuvette interne (4) , et en ce que la cuvette interne (4) présente, sur la partie de sa face externe (16) voisine de son bord libre (14), une saillie (17, 17') qui s'engage dans un évidement (13) en forme de rainure prévu sur la face interne (12) de la cuvette externe (2).

2. Cupule prothétique selon la revendication 1, caractérisée en ce que l'évidement (13) en forme de rainure à une section radiale sensiblement rectangulaire, et en ce que la saillie (17) de la cuvette interne (4) est rélisée avec une forme triangulaire, la dimension radiale (h) de la saillie (17) étant sensiblement égale à la profondeur radiale (t) de l'évidement (13), et la hauteur (H) de la saillie (17) étant inférieure à la hauteur (H') de l'évidement (13).

3. Cupule prothétique selon la revendication 1, caractérisée en ce que la saillie de la cuvette interne (4) s'engageant dans l'évidement (13) en forme de rainure de la cuvette externe (2) est constituée par un anneau (17') enchâssé dans la cuvette interne (4).

4. Cuvette de prothèse selon la revendication 3, caractérisée en ce que l'anneau (17'), présentant une section essentiellement rectangulaire, est chanfreiné sur son bord libre.

5. Cupule prothétique selon l'une quelconque des revendications 3 ou 4, caractérisé en ce que l'anneau (17') est muni d'une fente traversante s'étendant de façon sensiblement radiale.

6. Cupule prothétique selon l'une ou plusieurs des revendications 3 à 5, caractérisée en ce que l'anneau (17') est constitué en un métal précieux ou un alliage de métal précieux.

7. Cupule prothétique selon l'une ou plusieurs des revendications 3 à 5, caractérisée en ce que l'anneau (17') est constitué en titane.

8. Cupule prothétique selon l'une ou plusieurs des revendications 3 à 7, caractérisée en ce que l'anneau (17') est disposé dans un évidement (17'') en forme de rainure de la face extérieure (16) de la cuvette interne (4).

9. Cupule prothétique selon l'une ou plusieurs des revendications 3 à 8, caractérisée en ce que l'évidement (17'') en forme de rainure destiné à recevoir l'anneau (17') est formé par deux épaulements (19', 19'') en saillie sur la face extérieure (16) de la cuvette interne (4).

10. Cupule prothétique selon l'une ou plusieurs des revendications précédentes, caractérisée en ce qu'il est Prévu plusieurs couches tampon (7).

11. Cupule prothétique selon la revendication 10, caractérisée en ce qu'une couche d'appui intermédiaire est à chaque fois disposée entre deux couches tampon (7, 7) voisines l'une de l'autre.

12. Cupule prothétique selon la revendication 11, caractérisée en ce qu'une couche d'appui intermédiaire est constituée avec la même matière que la cuvette interne (4) et/ou la cuvette externe (2).

0 066 092

FIG.1

FIG.2

FIG.3

7

Fig.4

Fig.5

VI

19

7

FIG.7

VII                                    VII

19

7

FIG.6

Fig.8

Fig.9

FIG. 10

FIG. 11

FIG. 12